(19) Europäisches Patentamt
European Patent Office
Office européen des brevets

(11) Numéro de publication: **0 262 061 B1**

(12) **FASCICULE DE BREVET EUROPEEN**

(45) Date de publication de fascicule du brevet: **24.02.93**

(51) Int. Cl.[5]: **C07C 37/86**, C07C 37/00

(21) Numéro de dépôt: **87420251.8**

(22) Date de dépôt: **24.09.87**

(54) **Procédé de stabilisation des mélanges de chloration du phenol et/ou de chlorophenols.**

(30) Priorité: **25.09.86 FR 8613555**

(43) Date de publication de la demande:
**30.03.88 Bulletin 88/13**

(45) Mention de la délivrance du brevet:
**24.02.93 Bulletin 93/08**

(84) Etats contractants désignés:
**AT BE CH DE ES FR GB GR IT LI LU NL SE**

(56) Documents cités:
**EP-A- 0 036 378**
**FR-A- 1 317 387**
**FR-A- 1 320 738**
**US-A- 4 016 047**

(73) Titulaire: **RHONE-POULENC CHIMIE**
**25, quai Paul Doumer**
**F-92408 Courbevoie Cédex(FR)**

(72) Inventeur: **Desmurs, Jean La Jonquière**
**Route de Ternay Communay**
**F-69360 St-Symphorien d'Ozon(FR)**
Inventeur: **Ratton, Serge**
**Hameau de Belmont Vaulx Milieu**
**F-38090 Villefontaine(FR)**

(74) Mandataire: **Vignally, Noel et al**
**Rhône-Poulenc Interservices Service Brevets Chimie Centre de Recherches des Carrières B.P. 62**
**F-69192 Saint-Fons Cédex (FR)**

**EP 0 262 061 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé de stabilisation des mélanges réactionnels obtenus lors de la chloration du phénol et/ou de chlorophénols en tri-tétra- et pentachlorophénols.

Lors de la chloration du phénol, des monochlorophénols et des dichlorophénols en trichlorophénols, tétrachlorophénols ou pentachlorophénol, on obtient des mélanges de chloration qui sont colorés et qui évoluent dans le temps.

Ainsi, on a décrit dans US-A 4 016 047, un procédé pour éliminer les impuretés colorées qui consiste :

- à introduire dans le mélange contenant au moins un phénol polychloré, un inhibiteur de coloration choisi parmi les dérivés de phénol, les dérivés soufrés organiques et les phosphites organiques,
- à séparer le polychlorophénol, par distillation.

En ce qui concerne le problème de stockage, on observe notamment, même lors d'un stockage à froid, une augmentation du taux chlorophénoxyphénols et une évolution de la concentration de certains chlorophénols.

Lors de tels mélanges de chloration sont distillés, les produits de la distillation présentent également cette instabilité.

La demanderesse a mis en évidence la présence, dans ces mélanges de chloration, de cétones cycliques insaturées comportant une substitution gem-dichlorée, qui seraient à l'origine de l'évolution de ces mélanges.

La présente invention se propose de résoudre ce problème de l'instabilité des mélanges de chloration.

Plus précisément elle consiste en un procédé de stabilisation d'un mélange de chloration du phénol et/ou de chlorophénols, caractérisé en ce que l'on agite ledit mélange en présence d'au moins un composé réducteur accepteur de chlore.

Les composés réducteurs accepteurs de chlore que l'on peut utiliser dans la présente invention sont très variés.

Ces composés réducteurs accepteurs de chlore peuvent être notamment des phosphites organiques ; des phosphines ; des phosphinites ; des arsines ; des sulfures organiques ; des hydrures métalliques ; des borohydrures métalliques ou des dérivés de ces borohydrures métalliques tels que les cyanoborohydrures ou les alkoxyborohydrures ; certains métaux tels que le cuivre, l'étain, l'argent, le cobalt, le palladium, le platine, l'iridium, le germanium et le sélénium ou des dérivés de certain de ces métaux ; des phénols, diphénols ou triphénols.

Parmi ces composés réducteurs accepteurs de chlore, on peut distinguer différents groupes.

Le premier groupe particulièrement intéressant comprend notamment les dérivés du phosphore trivalent de formule générale (I) :

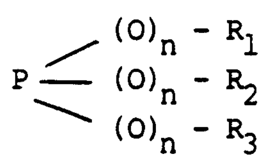

(I)

dans laquelle :

- $R_1$, $R_2$ et $R_3$, identiques ou différents, représentent :
  . un radical alkyle, linéaire ou ramifié, ayant 1 à 12 atomes de carbone ;
  . un radical alkyle, linéaire ou ramifié, ayant 1 à 12 atomes de carbone et comportant un à plusieurs atomes de chlore ;
  . un radical alkyle, comportant une ou plusieurs fonctions éthers, tel que notamment un ou plusieurs enchaînements oxyde d'éthylène et/ou oxyde de proylène ;
  . un radical phényle ;
  . un radical phényle comportant un ou plusieurs atomes de chlore ;
  . un radical phényle comportant 1 ou 2 substituants alkyles ayant 1 à 12 atomes de carbone ;
  . un radical phénylalkyle dont la partie aliphatique comporte 1 à 4 atomes de carbone et dont la partie cyclique peut comporter un ou plusieurs atomes de chlore ou 1 ou 2 radicaux alkyles ayant 1 à 12 atomes de carbone ;
  . un radical cycloalkyle, notamment cyclohexyle, éventuellement substitué par un ou plusieurs atomes de chlore ou 1 ou 2 radicaux alkyles ayant 1 à 12 atomes de carbone.
- un ou deux des radicaux $R_1$, $R_2$, et $R_3$ peut représenter un atome d'hydrogène;

- les symboles n, identiques ou différents, représentent 0 ou 1.

A titre d'exemples de composés de formule (I), on peut citer le phosphite de triphényle, les phosphites de tris(chlorophényle), les phosphites de tris(dichlorophényle), les phosphites de tris(trichlorophényle), le phosphite de triméthyle, le phosphite de triéthyle, le phosphite de tributyle, le phosphite de trioctyle, les phosphites de tris(nonylphényle), le phosphite de tris(ditertiobutyl-2,4 phényle), le phosphite de tribenzyle, le phosphite de tris(phénéthyle), le diphénylphosphinite de méthyle, le phosphite de tris(éthoxy-2 éthyle), le phosphite de tricyclohexyle, la triphénylphosphine, la diphénylméthylphosphine, la diphényléthylphosphine, la diéthylphénylphosphine, la tributylphosphine.

Ce premier groupe de composés réducteurs accepteurs de chlore comporte également les arsines telles que par exemple la triphénylarsine ; les triphénylarsines substituées par un ou des atomes de chlore ou 1 ou 2 groupements alkyles ayant 1 à 12 atomes de carbone ; les trisalkylarsines dont les groupements alkyles ont 1 à 12 atomes de carbone et peuvent éventuellement être substitués par des atomes de chlore ; les alkylphénylarsines telle que la diphényléthylarsine ; les arsénites telles que l'arsénite de triéthyle.

Enfin dans ce premier groupe de composés réducteurs accepteurs de chlore, on peut inclure également en raison de leur comportement analogue, les sulfures organiques, les thiols, les mercaptoacides et les acides thiocarboxyliques.

Ces sulfures organiques sont plus particulièrement les composés de formule générale (II) :

$R_5$ - S - $R_6$ (II)

dans laquelle $R_5$ et $R_6$ identiques ou différents, représentent :

- un radical phényle, éventuellement substitué par un ou plusieurs radicaux alkyles, alkoxy, hydroxyle ou atomes de chlore ;
- un radical alkyle ;
- un radical phénylalkyle, éventuellement substitué par un ou plusieurs radicaux alkyles, alkoxy, hydroxyle ou atomes de chlore ;
- un radical cyclopentyle ou cyclohexyle, éventuellement substitué par un ou pluieurs radicaux alkyles, alkoxy, hydroxyle ou atomes de chlore.

A titre d'exemples de tels sulfures organiques, on peut citer le thioanisole, le sulfure de diphényle, le sulfure de phényle et de chloro-4 phényle, le sulfure de di(chloro-4 phényle), le sulfure de di(hydroxy-2 ditertiobutyl-4,6 phényle), le sulfure de di(hydroxy-4 diméthyl-3,5 phényle), le sulfure de di(hydroxy-2 ditertiobutyl-3,5 phényle), le sulfure de phényle et de cyclohexyle, le sulfure de phényle et de butyle, le sulfure de chloro-4 phényle et d'hexyle, le sulfure de méthyl-4 phényle et de dodécyle, le sulfure de benzyle et d'octyle, le sulfure de méthoxy-4 phényle et d'éthyle, le sulfure de phényle et de stéaryle, le sulfure de dihexyle, le sulfure d'éthyle et d'hexyle, le sulfure de dioctyle, le sulfure de propyle et de méthyl-4 cyclohexyle, le sulfure de méthyle et de chloro-4 cyclohexyle.

Le thioanisole est un composé réducteur accepteur de chlore particulièrement efficace dans le procédé selon la présente invention.

Les thiols, les mercaptoacides et les acides thiocarboxyliques sont des composés connus. On se référera par exemples aux chapitres que leur sont consacrés dans l'Encyclopedia of Chemical Technology KIRK OTHMER, 3ème édition, volume 22, pages 946 à 964 ou dans HOUBEN-WEYL, Methoden Der Organischen Chemie (1955), volume IX, page 746 et suivantes.

On indiquera plus particulièrement les composés de formule générale (III) :

$R_7$ - SH (III)

dans laquelle $R_7$ représente :

- un radical alkyle linéaire ou ramifié, éventuellement substitué par un ou plusieurs groupements OH, groupements -COOH ou atomes de chlore et pouvant comporter dans leur chaîne un ou plusieurs atomes d'oxygène -O- ;
- un radical phényle, éventuellement substitué par un ou plusieurs radicaux alkyles, alkoxy, hydroxyle, carboxyle ou atomes de chlore ;
- un radical phénylalkyle, éventuellement substitué par un ou plusieurs radicaux alkyles, alkoxy, hydroxyle, carboxyle ou atomes de chlore ;
- un radical cyclopentyle ou cyclohexyle, éventuellement substitué par un ou plusieurs radicaux alkyles, alkoxy, hydroxyle ou carboxyle ou atomes de chlore ;
- un radical $R_7$ - CO - dans la formule duquel $R_7$ a les significations indiquées précédemment.

A titre d'exemples de tels thiols, mercaptoacides ou acides thiocarboxyliques, on peut citer le mercapto-2 éthanol, le toluènedithiol-3,4, le méthyl-2 propane-2 thiol, le thiphénol, l'acide thioacétique, l'acide thioglycolique, l'acide mercapto-2 succinique.

Les composés réducteurs accepteurs de chlore de ce premier groupe permettent la stabilisation des mélanges issus de la chloration du phénol et/ou de chlorophénols, en transformant tout ou partie des cétones cycliques insaturées gem-dichlorées, essentiellement en chlorophénols et peu ou pas en chlorophénoxyphénols.

Un deuxième groupe actif de composés réducteurs accepteurs de chlore comprend les hydrures métalliques, notamment de métaux alcalins, de métaux alcalino-terreux ou d'aluminium et métaux alcalins, les borohydrures de métaux alcalins ou des dérivés de borohydrures de métaux alcalins tels que les cyanoborohydrures et les alkoxyborohydrures.

On peut citer par exemple l'hydrure de sodium, l'hydrure de potassium, l'hydrure de lithium, l'hydrure de calcium, l'hydrure de magnésium, l'hydrure d'aluminium et de lithium, le borohydrure de sodium, le borohydrure de potassium, le cyanoborohydrure de sodium, le cyanoborohydrure de potassium, le triméthoxyborohydrure de sodium, le triéthoxyborohydrure de sodium, le triméthoxyborohydrure de potassium, le triéthoxyborohydrure de potassium, le diméthoxyborohydrure de sodium.

Ce deuxième groupe de composés réducteurs accepteurs de chlore comporte également certains métaux tels que le palladium, le platine, l'iridium, le cobalt, le cuivre, l'étain, l'argent sous leur forme métallique.

Il comporte également des dérivés de certains de ces métaux, généeralement à leur bas degré d'oxydation, notamment des sels compatibles avec le milieu réactionnel.

On peut citer par exemple le trichlorure de ruthénium et le chlorure stanneux.

Les composés réducteurs accepteurs de chlore de ce deuxième groupe permettent la stabilisation des mélanges issus de la chloration du phénol et/ou de chlorophénols, en transformant une partie des cétones cycliques insaturées gem-dichlorées en chlorophénols et une autre partie de ces cétones en chlorophénoxyphénols.

Un troisième groupe de composés réducteurs accepteurs de chlore comprend les phénols, les diphénols et les triphénols comportant ou non un ou plusieurs substituants alkyles ou alkoxy. Ces substituants alkyles ou alkoxy ont 1 à 4 atomes de carbone.

On peut citer par exemple le phénol, le méthyl-2 phénol, le méthyl-3 phénol, le méthyl-4 phénol, le méthoxy-2 phénol, le méthoxy-3 phénol, le méthoxy-4 phénol, l'éthoxy-2 phénol, l'éthoxy-3 phénol, l'éthoxy-4 phénol, le tertiobutyl-4 phénol, le pyrocatéchol, le résorcinol, l'hydroquinone, la méthyl-2 hydroquinone, la méthoxy-2 hydroquinone, le méthoxy-4 pyrocatéchol, la diméthoxy-2,6 hydroquinone, le naphtol-2, le naphtol-2, le dihydroxy-1,2 naphtalène, le dihydroxy-1,3 naphtalène, le dihydroxy-1,4 naphtalène, le dihydroxy-1,5 naphtalène, le dihydroxy-1,6 naphtalène, le dihydroxy-1,7 naphtalène, le dihydroxy-1,8 naphtalène, le dihydroxy-2,3 naphtaléne, le dihydroxy-2,6 naphtalène, le dihydroxy-2,7 naphtalène, le pyrogallol, le phloroglucinol, l'hydroxyquinol, la chloro-2 hydroquinone, la vitamine E.

Les composés réducteurs accepteurs de chlore de ce troisième groupe permettent la stabilisation des mélanges issus de la chloration du phénol et/ou de chlorophénols soit en transformant les cétones cycliques insaturées gem-dichlorées en chlorophénols, soit en réagissant avec les cétones cycliques pour conduite à des produits lourds du type phénoxyphénols ou biphényles chlorés.

Une variante du présent procédé consiste également dans l'addition au mélange issu de la chloration du phénol et/ou de chlorophénols de composés tels que le trichlorure de phosphore, l'acide phosphoreux ou l'acide hypophosphoreux par exemple, qui formeront in situ avec des chlorophénols présents, des phosphites de phényle chlorés, tels que ceux indiqués dans le premier groupe de composés réducteurs accepteurs de chlore.

Ces composés phosphorés peuvent également être ajoutés dans le mélange à traiter, avec un ou des phénols du troisième groupe de composés réducteurs accepteurs de chlore.

Les cétones cycliques insaturées gem-dichlorées sont essentiellement des cyclohexadiénones gem-dichlorées comportant par ailleurs 1, 2, 3 ou 4 autres atomes de chlore sur différents atomes de carbone du cycle et des cyclohexénones gem-dichlorées comportant également par ailleurs 1 à 6 autres atomes de chlore sur différents atomes de carbone du cycle.

Ce sont d'une part les dichloro-4,4 cyclohexadiène-2,5 ones et les dichloro-6,6 cyclohexadiène-2,4 ones comportant en outre 1 à 4 atomes de chlore.

A titre d'exemples des principales cyclohexadiénones gem-dichlorées, on peut citer:
la dichloro-6,6 cyclohexadiène-2,4 one,
le dichloro-4,4 cyclohexadiène-2,5 one,
le tétrachloro-2,4,4,6 cyclohexadiène-2,5 one,

la tétrachloro-2,4,6,6 cyclohexadiène-2,4 one,
la pentachloro-2,3,4,4,6 cyclohexadiène-2,5 one,
la pentachloro-2,4,5,6,6 cyclohexadiène-2,4 one,
la pentachloro-2,3,4,6,6 cyclohexadiène-2,4 one,
l'hexachloro-2,3,4,4,5,6 cyclohexadiène-2,5 one,
et l'hexachloro-2,3,4,5,6,6 cyclohexadiène-2,4 one.

Ce sont d'autre part les dichloro-2,2, cyclohexène-3 ones, les dichloro-6,6 cyclohexène-2 ones, les dichloro-4,4 cyclohexène-2 ones, les dichloro-6,6 cyclohexène-3 ones comportant en outre 1 à 6 atomes de chlore.

A titre d'exemples des principales cyclohexènones gem-dichlorées, on peut citer :
la pentachloro-2,4,5,6,6 cyclohexène-2 one,
l'hexachloro-2,4,4,5,6,6 cyclohexène-2 one,
l'hexachloro-2,2,4,5,6,6 cyclohexène-3 one,
l'heptachloro-2,4,4,5,5,6,6 cyclohexène-2 one,
l'heptachloro-2,2,3,4,5,6,6 cyclohexène-3 one,
l'heptachloro-2,3,4,4,5,5,6 cyclohexène-2 one,
l'heptachloro-2,3,4,4,5,6,6 cyclohexène-2 one,
l'octachloro-2,3,4,4,5,5,6,6 cyclohexène-2 one,
et l'octachloro-2,2,3,4,5,5,6,6 cyclohexène-3 one.

La température à laquelle sont agités le mélange issue de la chloration du phénol et/ou du chlorophénols et le ou les composés réducteurs accepteurs de chlore varie dans de larges limites, par exemple entre 20 ° C et 200 ° C.

De préférence cependant, pour avoir une bonne réaction, la température sera de 40 ° C à 180 ° C et le plus souvent de 60 ° C à 150 ° C.

La durée du traitement est très variable en fonction de la température, de la quantité de cétones cycliques gem-dichlorées contenues dans le mélange et du composé réducteur accepteur de chlore utilisé. Elle peut varier par exemple de quelques minutes à plusieurs dizaines d'heures.

En général, elle se situe entre 1 heure et 15 heures, sans que ces nombres aient une valeur critique.

La quantité de composé réducteur accepteur de chlore dépend bien évidemment de la teneur en cétones cycliques gem-dichlorées du mélange et de leur nature. Ces cétones sont généralement dosées dans le mélange par chromatographie en phase liquide à double détection ; un détecteur à ultra-violets pour l'ensemble des composés du mélange et un détecteur électrochimique spécifiquement pour les cétones cycliques gem-dichlorées ou par un dosage global en électrochimie.

Pour avoir une bonne stabilisation des mélanges de chloration, il faut introduire une quantité molaire de composé réducteur accepteur de chlore au moins égale à la quantité molaire des cétones cycliques gem-dichlorées.

Comme il n'est pas toujours aisé de déterminer avec précision la nature des différentes cétones cycliques insaturées gem-dichlorées des mélanges de chloration, il est préférable d'introduire un excès molaire de composé réducteur accepteur de chlore.

Les exemples qui suivent illustrent la présente invention.

EXEMPLES 1 à 6

Dans un réacteur en verre de 10 cm3 avec agitation, on charge:

| | |
|---|---|
| - trichloro-2,4,6 phénol : | 1,97 g (0,010 mole) |
| - tétrachloro-2,4,4,6 cyclohexadiène-2,5 one : | 0,23 g (0,001 mole) |
| - composé réducteur accepteur de chlore : | 0,0025 mole. |

On chauffe le mélange sous agitation pendant 8 heures à 75 ° C.

On analyse la masse réactionnelle par chromatographie en phase liquide, à l'aide d'une double détection UV (totalité des composés chlorés) et ampérométrie (cétones cycliques insaturées gem-dichlorées).

On effectue dans les mêmes conditions un essai térmoin avec les mêmes charges à l'exception du composé réducteur accepteur de chlore.

Le tableau (I) ci-après indique la nature et la quantité de composé réducteur accepteur de chlore utilisé, ainsi que les résultats des anayses effectuées après le traitement.

| ESSAIS | Réducteur accepteur de chlore Poids en grammes | TT % de la chlorocyclohexadiènone | RT % en tétrachloro-2,3,4,6 phénol | Poids de D-4,6 PP en g | Poids de D-2,6 PP en g |
|---|---|---|---|---|---|
| Témoin A | Néant | 4,8 | 19,8 | 3,52 x $10^{-2}$ | 2,2 x $10^{-2}$ |
| Exemple 1 | Phosphite de triphényle : 0,77 | 100 | 1,15 | 0 | 0 |
| Exemple 2 | Triphénylphosphine : 0,65 | 100 | 1,30 | 0 | 0 |
| Exemple 3 | Hydrure d'Al et de Li : 0,09 | 94,0 | 8,4 | 9,62 x $10^{-2}$ | 8,24 x $10^{-2}$ |
| Exemple 4 | Borohydrure de Na : 0,09 | 86,1 | 0,75 | 2,3 x $10^{-2}$ | 1,37 x $10^{-2}$ |
| Exemple 5 | Cuivre métal : 0,16 | 100 | 0,70 | 10,86 x $10^{-2}$ | 10,3 x $10^{-2}$ |
| Exemple 6 | Chlorure stanneux : 0,47 | 99,6 | 0 | 2,7 x $10^{-2}$ | 15,2 x $10^{-2}$ |

Tableau (I)

TT = taux de transformation ;
RT = rendement par rapport à la chlorocyclohexadiènone transformée ;
D-4,6 PP = Dichloro-4,6 (trichloro-2',4',6' phénoxy)-2 phénol
D-2.6 PP = Dichloro-2.6 (trichloro-2',4',6' phénoxy)-4 phénol.

EXEMPLES 7 à 9

Dans un réacteur en verre de 10 cm3, avec agitation on charge :

| | |
|---|---|
| - trichloro-2,4,6 phénol :<br>- hexachloro-2,3,4,4,5,6 cyclohexadiène-2,5 one :<br>- composé réducteur accepteur de chlore : | 1,97 g (0,010 mole)<br>0,30 g (0,001 mole)<br>0,0025 mole. |

On opère comme dans les exemples 1 à 6 : 8 heures à 75 °C.

Le tableau II ci-après indique la nature et la quantité de composé réducteur accepteur de chlore utilisé, ainsi que les résultats des analyses effectuées après le traitement.

| ESSAIS | Réducteur accepteur de chlore Poids en grammes | TT % de l'hexachlorocyclohexadiènone | RT % en pentachlorophénol | Poids de CCPP en g | Poids de TCCD |
|---|---|---|---|---|---|
| Témoin B | Néant | 4,9 | 0 | 0 | 0 |
| Exemple 7 | Phosphite de triphényle : 0,77 | 100 | 100 | $0,45 \times 10^{-2}$ | 0 |
| Exemple 8 | Triphénylphosphine : 0,66 | 100 | 100 | 0 | 0 |
| Exemple 9 | Tributylphosphine : 0,51 | 61,0 | 84,9 | $2,06 \times 10^{-2}$ | $2,39 \times 10^{-2}$ |

**Tableau II**

CCPP = mélange de chloro(chlorophénoxy)phénols
TCCD = tétrachloro-2,4,4,6 cyclohexadiène-2,5 one.

EXEMPLES 10 à 19

Dans un réacteur en verre de 10 cm3 avec agitation, on charge :

| | |
|---|---|
| - trichloro-2,4,6 phénol :<br>- tétrachloro-2,4,4,6 cyclohexadiène-2,5 one :<br>- composé réducteur accepteur de chlore : | 1,97 g (0,010 mole)<br>0,23 g (0,001 mole)<br>0,0025 mole. |

On chauffe le mélange sous agitation pendant 8 heures à 75 ° C.

On analyse la masse réactionnelle par chromatographie en phase liquide, à l'aide d'une double détection UV (totalité des composés chlorés) et ampérométrie (cétones cycliques insaturées gem-dichlorées).

Le tableau (III) ci-après indique la nature et la quantité de composé réducteur accepteur de chlore utilisé, ainsi que les résultats des analyses effectuées après le traitement.

**Tableau (III)**

| EXEMPLES | Réducteur accepteur de chlore Poids en grammes | TT % de la chlorocyclohexadiènone | RT % en tétrachloro-2,3,4,6 phénol | Poids de D-4,6 PP en g | Poids de D-2,6 PP en g |
|---|---|---|---|---|---|
| Exemple 10 | Phénol : 0,24 | 100 | 0 | 0 | 0 |
| Exemple 11 | Thioanisole : 0,31 | 100 | 0 | 0 | 0 |
| Exemple 12 | Résorcinol : 0,27 | 100 | 0 | 0 | 0 |
| Exemple 13 | Hydroquinone : 0,27 | 100 | 0 | 0 | 0 |
| Exemple 14 | Pyrocatéchol : 0,27 | 100 | 0 | 0 | 0 |
| Exemple 15 | Pyrogallol : 0,31 | 100 | 0,2 | 0 | 0 |
| Exemple 16 | Chloro-2 hydroquinone : 0,38 | 98,0 | 0 | 0 | 0 |

.../...

Tableau (III) - suite

| EXEMPLES | Réducteur accepteur de chlore Poids en grammes | TT % de la chlorocyclohexadiénone | RT % en tétrachloro-2,3,4,6 phénol | Poids de D-4,6 PP en g | Poids de D-2,6 PP en g |
|---|---|---|---|---|---|
| Exemple 17 | $RuCl_3$ : 0,52 | 70,0 | 0 | 0 | 0 |
| Exemple 18 | Vitamine E : 0,115 | 100 | 0 | 0 | 0 |
| Exemple 19 | Triméthylhydroquinone : 0,38 | 100 | 0 | 0 | 0 |

TT = taux de transformation ;
RT = rendement par rapport à la chlorocylohexadiénone transformée ;
D-4,6 PP = dichloro-4,6 (trichloro-2',4',6' phénoxy)-2 phénol ;
D-2,6 PP = dichloro-2,6 (trichloro-2',4',6' phénoxy)-4 phénol.

EXEMPLES 20 à 24

Dans le réacteur décrit dans les exemples 1 à 6, on charge :

| | |
|---|---|
| - tétrachloro-2,4,4,6 cyclohexadiène-2,5 one :<br>- composé phénolique :<br>- composé réducteur accepteur de chlore : | 0,23 g (0,001 mole)<br>0,010 mole<br>0,0025 mole. |

On opère comme indiqué pour les exemples 1 à 6.
Durée des essais : 8 heures à 70 ° C.
Le tableau IV ci-après indique la nature du composé phénolique et due composé réducteur utilisés, ainsi que les résultats des analyses effectuées après le traitement.

| ESSAIS | Composé phénolique Réducteur accepteur de chlore | TT % de la chlorocyclohexadiènone | RT % en trichloro-2,4,6 phénol | RT % en tétrachloro-2,3,4,6 phénol | Poids de chlorophénoxyphénols en g |
|---|---|---|---|---|---|
| Témoin C | trichloro-2,4,6 phénol | 8 | - | 0 | 0 |
| Exemple 20 | phénol triphénylphosphine | 100 | 98,1 | 1,9 | 0 |
| Exemple 21 | orthochlorophénol triphénylphosphine | 100 | 91,8 | 0,3 | 0 |
| Exemple 22 | dichloro-2,4 phénol triphénylphosphine | 100 | 100 | 0 | 0 |
| Exemple 23 | parachlorophénol thioanisole | 100 | 70,4 | 0 | 0 |
| Exemple 24 | trichloro-2,4,6 phénol vitamine E | 100 | - | 0 | 0 |

**Tableau (IV)**

EXEMPLES 25 à 31

Dans le réacteur décrit dans les exemples 1 à 6, on charge :

| | |
|---|---|
| - pentachloro-2,3,4,4,6 cyclohexadiène-2,5 one :<br>- composé phénolique :<br>- composé réducteur accepteur de chlore : | 0,265 g (0,001 mole)<br>0,010 mole<br>0,0025 mole. |

On opère comme indiqué dans les exemples 1 à 6.

Durée des essais : 8 heures à 70 ° C.

Le tableau (V) ci-après indique la nature du composé phénolique et due composé réducteur utilisés, ainsi que les résultats des analyses effectuées après le traitement.

| ESSAIS | Composé phénolique Réducteur accepteur de chlore | TT % de la chlorocyclo-hexadiènone | RT % en tétra-chloro-2,3,4,6 phénol | RT % en penta-chlorophénol | Poids de chlorophénoxy-phénols en g |
|---|---|---|---|---|---|
| Exemple 25 | phénol triphénylphosphine | 100 | 96,1 | 0,1 | 0 |
| Exemple 26 | orthochlorophénol triphénylphosphine | 100 | 100 | 0,1 | 0 |
| Exemple 27 | parachlorophénol triphénylphosphine | 100 | 100 | 0,1 | 0 |
| Exemple 28 | dichloro-2,4 phénol triphénylphosphine | 100 | 99,0 | 0 | 0 |
| Exemple 29 | trichloro-2,4,6 phénol triphénylphosphine | 100 | 82,4 | 0 | 0 |
| Exemple 30 | tétrachloro-2,3,4,6 phénol triphénylphosphine | 100 | - | 0 | 0 |
| Exemple 31 | trichloro-2,4,6 phénol phosphite de triphényle | 100 | 99,1 | 0 | 0 |

**Tableau (V)**

EXEMPLES 32 à 37

Dans le réacteur décrit dans les exemples 1 à 6, on charge :

| | |
|---|---|
| - hexachloro-2,3,4,4,5,6 cyclohexanediène-2,5 one :<br>- composé phénolique :<br>- composé réducteur accepteur de chlore : | 0,30 g (0,001 mole)<br>0,010 mole<br>0,0025 mole. |

On opère comme indiqué dans les exemples 1 à 6.
Durée des essais : 8 heures à 70 ° C.
Le tableau (VI) ci-après indique la nature du composé phénolique et du composé réducteur utilisés, ainsi que les résultats des analyses effectuées après le traitement.

| ESSAIS | Composé phénolique Réducteur accepteur de chlore | TT % de la chlorocyclohexadiènone | TT % en pentachlorophénol | Poids de chlorophénoxyphénols en g |
|---|---|---|---|---|
| Témoin D | trichloro-2,4,6 phénol<br>- | 2 | 0 | 0 |
| Exemple 32 | phénol<br>triphénylphosphine | 100 | 100 | 0 |
| Exemple 33 | orthochlorophénol<br>triphénylphosphine | 100 | 100 | 0 |
| Exemple 34 | dichloro-2,6 phénol<br>triphénylphosphine | 100 | 94,1 | 0 |
| Exemple 35 | dichloro-2,4 phénol<br>triphénylphosphine | 100 | 97,0 | 0 |
| Exemple 36 | tétrachloro-2,3,4,6 phénol<br>triphénylphosphine | 100 | 96,0 | 0 |
| Exemple 37 | pentachlorophénol<br>triphénylphosphine | 100 | - | 0 |

Tableau (VI)

EXEMPLES 38 à 40

Traitement d'un brut de chloration.

Dans un tricol muni d'un réfrigérant, d'une agitation centrale à pales et d'un tube plongeant pour l'introduction du chlore, on charge 100 g de dichloro-2,4 phénol (613 mmol) et 0,1 g de diisopropylamine. Après avoir chauffé le mélange réactionnel à 70 ° C, on introduit en 3 heures 30 minutes 15,2 litres de $Cl_2$ - (675 mmol). Après refroidissement, les analyses par chromatographie en phase gazeuse et chromatographie en phase liquide munie d'une double détection U.V. et électrochimique conduit aux résultats suivants :

- tétrachloro-2,4,4,6 cyclohexadiènone :
  1,5 % → 7,9 mmol → RT = 1,3 %
- trichloro-2,4,6 phénol :
  96,1 % → 583,3 mmol → RT = 96,8%
- dichloro-2,4 phénol :
  0,05 % → 0,4 mmol → TT = 99,9
- tétrachloro-2,3,4,6 phénol :
  1,4 % → 7,4 mmol → RT = 1,2 %

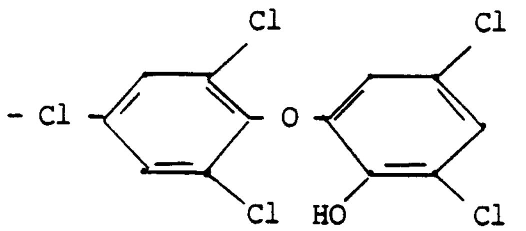

0,45 % → 1,5 mmol → RT = 0,5 %

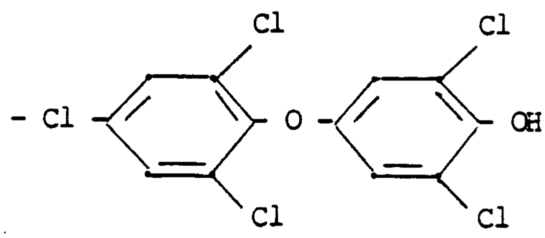

0,3 % → 1 mmol → RT = 0,3 %

Exemple 38 : traitement avec la triphénylphosphine

A 5,1 g de la masse réactionnelle (0,33 mmol) de tétrachlorocyclohexadiènone), on ajoute 0,2 g de triphénylphosphine (0,64 mmol), puis on chauffe le mélange 1 heure 30 minutes à 70 ° C. La solution se décolore rapidement au cours du chauffage. Après refroidissement, on ne détecte plus en analyse de tétrachloro-2,4,4,6 cyclohexadiènone.

Exemple 39 : Traitement avec l'acide hypophosphoreux

A 5,1 g de la masse réactionnelle (0,33 mmol de tetrachlorocyclohexadiènone), on ajoute 0.2 g d'acide hypophosphoreux à 50 % (1,5 mmol) ; ce mélange est chauffé 96 heures à 70 ° C. Après refroidissement, on ne détecte plus de tétrachloro-2,4,4,6 cyclohexadiènone.

Exemple 40 : Traitement avec $PCl_3$

A 83,8 g de la masse réactionnelle brute (5,4 mmol de tétrachloro-2,4,4,6 cyclohexadiène-2,4 one), on ajoute 3,35 g de $PCl_3$ (24,4 mmol), puis on chauffe à 70 ° C durant 1 heure 20 minutes. Après refroidissement, on ne détecte plus de tétrachlorocyclohexadiènone en analyse.

EXEMPLES 41 et 42

Dans un flacon en verre de 10 cm3, muni d'un bouchon étanche, on introduit :

| | |
|---|---|
| - tétrachloro-2,4,4,6 cyclohexadiène-2,5 one :<br>- trichloro-2,4,6 phénol :<br>- thiol : | 0,23 g (0,001 mole)<br>1,97 g (0,010 mole)<br>0,0025 mole. |

Le mélange est chauffé jusqu'à fusion, puis il est placé dans une étuve à 70 ° C pendant 8 heures.

Par les analyses décrites dans les exemples 1 à 6, on obtient les résultats suivants :

Exemple 41 avec mercapto-2 éthanol (0,20 g)

| | |
|---|---|
| - TT de la tétrachloro-2,4,4,6 cyclohexadiène-2,5 one : | 100 % |
| - RT en phénoxyphénols : | 0% |
| - RT en trichloro-2,4,6 phénol : | 51,4 % |

Exemple 42 avec toluènedithiol-3,4 (0,39 g)

| | |
|---|---|
| - TT de la tétrachloro-2,4,4,6 cyclohexadiène-2,5 one : | 100 % |
| - RT en phénoxyphénols : | 0 % |
| - RT en trichloro-2,4,6 phénol : | 100 % |

EXEMPLES 43 à 45

Dans un flacon en verre de 10 cm3, muni d'un bouchon étanche, on introduit :

| | |
|---|---|
| - tétrachloro-2,4,4,6 cyclohexadiène-2,5 one : | 0,23 g (0,001 mole) |
| - phénol : | 0,94 g (0,010 mole) |
| - thiol : | 0,0025 mole. |

Le mélange est chauffé jusqu'à fusion, puis il est placé dans une étuve à 70 ° C pendant 8 heures. On obtient les résultats suivants :

| EXEMPLES | Tniol utilisé | TT% de la tétrachloro-2,4,4,6 cyclohexadiène-2,5 one | RT % en trichloro-2,4,6 phénol |
|---|---|---|---|
| Exemple 43 | Acide thioacétique | 100 | 35,8 |
| Exemple 44 | méthyl-3 propane-2 thiol | 100 | 88,6 |
| Exemple 45 | thiophénol | 100 | 31,2 |

## Revendications

**1.** Procédé de stabilisation d'un mélange issu de la chloration du phénol et/ou de chlorophénols, caractérisé en ce que l'on agite ledit mélange en présence d'au moins un composé réducteur accepteur de chlore.

**2.** Procédé selon la revendication 1, caractérisé en ce que le composé réducteur accepteur de chlore est choisi parmi les phosphites organiques ; les phosphines ; les phosphinites ; les arsines ; les sulfures organiques ; les thiols ; les acides thiocarboxyliques ; les mercaptoacides ; les hydrures métalliques ; les borohydrures métalliques ; les cyanoborohydrures métalliques ; les alkoxyborohydrures ; certains métaux tels que le cuivre, l'étain, l'argent, le colbalt, le palladium, le platine, l'iridium, le germanium et le sélénium ou des dérivés de certains de ces métaux ; les phénols, diphénols et triphénols.

**3.** Procédé selon l'une des revendications 1 ou 2, caractérisé en ce que le composé réducteur accepteur de chlore est un dérivé du phosphore trivalent de formule générale (I) :

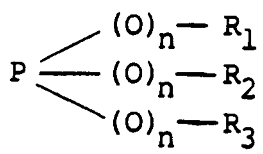

(I)

dans laquelle :

- $R_1$, $R_2$ et $R_3$, identiques ou différents, représentent :
  . un radical alkyle, linéaire ou ramifié, ayant 1 à 12 atomes de carbone ;
  . un radical alkyle, linéaire ou ramifié, ayant 1 à 12 atomes de carbone et comportant un à plusieurs atomes de chlore ;
  . un radical alkyle, comportant une ou plusieurs fonctions éthers, tel que notamment un ou plusieurs enchaînements oxyde d'éthylène et/ou oxyde de propylène ;
  . un radical phényle ;
  . un radical phenyle comportant un ou plusieurs atomes de chlore ;
  . un radical phényle comportant 1 ou 2 substituants alkyles ayant 1 à 12 atomes de carbone ;
  . un radical phenylalkyle dont la partie aliphatique comporte 1 à 4 atomes de carbone et dont la partie cyclique peut comporter un ou plusieurs atomes de chlore ou 1 ou 2 radicaux alkyles ayant 1 à 12 atomes de carbone ;
  . un radical cycloalkyle, notamment cylohexyle, éventuellement substitué par un ou plusieurs atomes de chlore ou 1 ou 2 radicaux alkyles ayant 1 à 12 atomes de carbone.
- un ou deux des radicaux $R_1$, $R_2$ et $R_3$ peut représenter un atome d'hydrogène ;
- les symboles n, identiques ou différents, représentent 0 ou 1.

**4.** Procédé selon la revendication 3, caractérisé en ce que le dérivé du phosphore trivalent de formule (I) est choisi parmi le phosphite de triphényle, les phosphites de tris(chlorophényle), les phosphites de tris(dichlorophényle), les phosphites de tris(trichlorophényle), le phosphite de triméthyle, le phosphite de triéthyle, le phosphite de tributyle, le phosphite de trioctyle, les phosphites de tris(nonylphényle) le phosphite de tris(ditertiobutyl-2,4 phènyle) le phosphite de tribenzyle, le phosphite de tris(phénéthyle), le diphénylphosphinite de méthyle, le phosphite de tris(éthoxy-2 éthyle), le phosphite de tricyclohexyle, la triphénylphosphine, la diphénylméthylphosphine, la diphényléthylphosphine, la diéthylphénylphosphine, la tributylphosphine.

**5.** Procédé selon l'une des revendications 1 ou 2, caractérisé en ce que le composé réducteur accepteur de chlore est choisi parmi les sulfures organiques de formule générale (II) :

$R_5$ - S - $R_6$ (II)

dans laquelle $R_5$ et $R_6$, identiques ou différents, représentent :

- un radical phényle, éventuellement substitué par un ou plusieurs radicaux alkyles, alkoxy, hydroxyle ou atomes de chlore ;

- un radical alkyle ;
- un radical phénylalkyle, éventuellement substitué par un ou plusieurs radicaux alkyles, alkoxy, hydroxyle ou atomes de chlore ;
- un radical cyclopentyle ou cyclohexyle, éventuellement substitué par un ou plusieurs radicaux alkyles, alkoxy, hydroxyle ou atomes de chlore.

**6.** Procédé selon la revendication 5, caractérisé en ce que le composé réducteur accepteur de chlore est choisi parmi le thioanisole, le sulfure de diphényle, le sulfure de phényle et de chloro-4 phényle, le sulfure de di(chloro-4 phényle), le sulfure de di(hydroxy-2 ditertiobutyl-4,6 phényle), le sulfure de di-(hydroxy-4 diméthyl-3,5 phényle), le sulfure de di(hydroxy-2 ditertiobutyl-3,5 phényle), le sulfure de phényle et de cyclohexyle, le sulfure de phényle et de butyle, le sulfure de chloro-4 phényle et d'hexyle, le sulfure de méthyl-4 phényle et de dodécycle, le sulfure de benzyle et d'octyle, le sulfure de méthoxy-4 phényle et d'éthyle, le sulfure de phényle et de stéaryle, le sulfure de dihexyle, le sulfure d'éthyle et d'hexyle, le sulfure de dioctyle, le sulfure de propyle et de méthyl-4 cyclohexyle, le sulfure de méthyle et de chloro-4 cyclohexyle.

**7.** Procédé selon les revendications 5 ou 6, caractérisé en ce que le composé réducteur accepteur de chlore utilisé est le thioanisole.

**8.** Procédé selon l'une des revendications 1 ou 2, caractérisé en ce que le composé réducteur accepteur de chlore est choisi parmi les thiols, les mercaptoacides et les acides thiocarboxyliques de formule générale (III) :

$R_7$ - SH (III)

dans laquelle $R_7$ représente :

- un radical alkyle linéaire ou ramifié, éventuellement substitué par un ou plusieurs groupements OH, groupements -COOH ou atomes de chlore et pouvant comporter dans leur chaîne un ou plusieurs atomes d'oxygène -O- ;
- un radical phényle, éventuellement substitué par un ou plusieurs radicaux alkyles, alkoxy, hydroxyle, carboxyle ou atomes de chlore ;
- un radical phénylalkyle, éventuellement substitué par un ou plusieurs radicaux alkyles, alkoxy, hydroxyle, carboxyle ou atomes de chlore ;
- un radical cyclopentyle ou cyclohexyle, éventuellement substitué par un ou plusieurs radicaux alkyles, alkoxy, hydroxyle ou carboxyle ou atomes de chlore ;
- un radical $R_7$ - CO - dans la formule duquel $R_7$ a les significations indiquées précédemment.

**9.** Procédé selon la revendication 8, caractérisé en ce que le composé réducteur accepteur de chlore utilisé est choisi parmi le mercapto-2 éthanol, le toluènedithiol-3,4, le méthyl-2 propane-2 thiol, le thiophénol, l'acide thioacétique, l'acid thioglycolique, l'acide mercapto-2 succinique.

**10.** Procédé selon l'une des revendicaitons 1 ou 2, caractérisé en ce que le composé réducteur accepteur de chlore est choisi parmi les hydrures de métaux alcalins, de métaux alcalino-terreux ou d'aluminium et de métaux alcalins ; les borohydrures de métaux alcalins ; les cyanoborohydrures de métaux alcalins et les alkoxyborohydrures de métaux alcalins.

**11.** Procédé selon la revendication 10, caractérisé en ce que le composé réducteur accepteur de chlore est choisi parmi l'hydrure de sodium, l'hydrure de potassium, l'hydrure de lithium, l'hydrure de calcium, l'hydrure de magnésium, l'hydrure d'aluminium et de lithium, le borohydrure de sodium, le borohydrure de potassium, le cyanoborohydrure de sodium, le cyanoborohydrure de potassium, le triméthoxyborohydrure de sodium, le triéthoxyborohydrure de sodium, le triméthoxyborohydrure de potassium, le triéthoxyborohydrure de potassium, le diméthoxyborohydrure de sodium.

**12.** Procédé selon l'une des revendications 1 ou 2 caractérisé en ce que le composé réducteur accepteur de chlore est choisi parmi le cuivre, le chlorure stanneux, le trichlorure de ruthénium.

**13.** Procédé selon l'une des revendications 1 ou 2 caractérisé en ce que le composé réducteur accepteur de chlore est choisi parmi les phénols, le diphénols et les triphénols et ces composés comportant un

ou plusieurs substituants alkyles ou alkoxy ayant 1 à 4 atomes de carbone.

**14.** Procédé selon la revendication 13, caractérisé en ce que le composé réducteur accepteur de chlore est choisi parmi le phénol, le méthyl-2 phénol, le méthyl-3 phénol, le méthyl-4 phénol, le méthoxy-2 phénol, le méthoxy-3 phénol, le méthoxy-4 phénol, l'éthoxy-2 phénol, l'éthoxy-3 phénol, l'éthoxy-4 phénol, le tertiobutyl-4 phénol, le pyrocatéchol, le résorcinol, l'hydroquinone, la méthyl-2 hydroquinone, la méthoxy-2 hydroquinone, le méthoxy-4 pyrocatéchol, la diméthoxy-2,6 hydroquinone, le naphtol-1, le naphtol-2, le dihydroxy-1,2 naphtalène, le dihydroxy-1,3 naphtalène, le dihydroxy-1,4 naphtalène, le dihydroxy-1,5 naphtalène, le dihydroxy-1,6 naphtalène, le dihydroxy-1,7 naphtalène, le dihydroxy-1,8 naphtalène, le dihydroxy-2,3 naphtalène, le dihydroxy-2,6 naphtalène, le dihydroxy-2,7 naphtalène, le pyrogallol, le phloroglucinol, l'hydroxyquinol, la chloro-2 hydroquinone, la vitamine E.

**15.** Procédé selon l'une des revendications 1, 13 et 14, caractérisé en ce que l'on introduit dans le mélange à traiter du trichlorure de phosphore, de l'acide phosphoreux ou de l'acide hypophosphoreux.

**16.** Procédé selon l'une des revendications 1 à 15, caractérisé en ce que l'on opère à une température de 20° C à 200° C et de préférence de 60 à 150° C.

**17.** Procédé selon l'une des revendications 1 à 16, caractérisé en ce que l'on utilise une quantité molaire de composé réducteur accepteur de chlore au moins égale à la quantité molaire des cétones cycliques gem-dichlorées du mélange à traiter et de préférence un excès molaire de composé réducteur accepteur de chlore.

## Claims

**1.** Process for the stabilisation of a mixture resulting from the chlorination of phenol and/or chlorophenols, characterised in that the said mixture is stirred in the presence of at least one chlorine-accepting reducing compound.

**2.** Process according to Claim 1, characterised in that the chlorine-accepting reducing compound is chosen from organic phosphites; phosphines; phosphinites; arsines; organic sulphides; thiols; thiocarboxylic acids; mercapto acids; metal hydrides; metal borohydrides; metal cyanoborohydrides; alkoxyborohydrides; certain metals such as copper, tin, silver, cobalt, palladium, platinum, iridium, germanium and selenium or derivatives of certain of these metals; phenols, diphenols and triphenols.

**3.** Process according to either of Claims 1 and 2, characterised in that the chlorine-accepting reducing compound is a derivative of trivalent phosphorus of general formula (I):

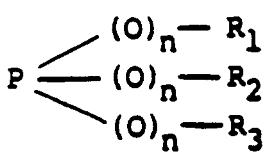

(I)

in which:

- $R_1$, $R_2$ and $R_3$, which are identical or different, represent:
  - a linear or branched alkyl radical having 1 to 12 carbon atoms;
  - a linear or branched alkyl radical having 1 to 12 carbon atoms and containing one or more chlorine atoms;
  - an alkyl radical, containing one or more ether functional groups, such as, in particular, one or more ethylene oxide and/or propylene oxide linkages;
  - a phenyl radical;
  - a phenyl radical containing one or more chlorine atoms;
  - a phenyl radical containing 1 or 2 alkyl substituents having 1 to 12 carbon atoms;
  - a phenylalkyl radical, the aliphatic part of which contains 1 to 4 carbon atoms and the cyclic part of which can contain one or more chlorine atoms or 1 or 2 alkyl radicals having 1 to 12

carbon atoms;
- a cycloalkyl radical, in particular cyclohexyl, optionally substituted by one or more chlorine atoms or 1 or 2 alkyl radicals having 1 to 12 carbon atoms,
- one or two of the radicals $R_1$, $R_2$ and $R_3$ can represent a hydrogen atom;
- the symbols n, which are identical or different, represent 0 or 1.

**4.** Process according to Claim 3, characterised in that the derivative of trivalent phosphorous of formula (I) is chosen from triphenyl phosphite, tris(chlorophenyl) phosphites, tris(dichlorophenyl) phosphites, tris-(trichlorophenyl) phosphites, trimethyl phosphite, triethyl phosphite, tributyl phosphite, trioctyl phosphite, tris(nonylphenyl) phosphites, tris(2,4-di(tert-butyl)phenyl) phosphite, tribenzyl phosphite, tris-(phenethyl) phosphite, methyl diphenylphosphinite, tris(2-ethoxyethyl) phosphite, tricyclohexyl phosphite, triphenylphosphine, diphenylmethylphosphine, diphenylethylphosphine, diethylphenylphosphine or tributylphosphine.

**5.** Process according to either of Claims 1 and 2, characterised in that the chlorine-accepting reducing compound is chosen from the organic sulphides of general formula (II):

$R_5$ - S - As (II)

in which $R_5$ and $R_6$, which are identical or different, represent:
- a phenyl radical, optionally substituted by one or more alkyl, alkoxy or hydroxyl radicals or chlorine atoms;
- an alkyl radical;
- a phenylalkyl radical, optionally substituted by one or more alkyl, alkoxy or hydroxyl radicals or chlorine atoms;
- a cyclopentyl or cyclohexyl radical, optionally substituted by one or more alkyl, alkoxy or hydroxyl radicals or chlorine atoms.

**6.** Process according to Claim 5, characterised in that the chlorine-accepting reducing compound is chosen from thioanisole, diphenyl sulphide, phenyl 4-chlorophenyl sulphide, di(4-chlorophenyl) sulphide, di(2-hydroxy-4,6-di(tert-butyl)phenyl) sulphide, di(4-hydroxy-3,5-dimethylphenyl) sulphide, di(2-hydroxy-3,5-di(tert-butyl)phenyl) sulphide, phenyl cyclohexyl sulphide, phenyl butyl sulphide, 4-chlorophenyl hexyl sulphide, 4-methylphenyl dodecyl sulphide, benzyl octyl sulphide, 4-methoxyphenyl ethyl sulphide, phenyl stearyl sulphide, dihexyl sulphide, ethyl hexyl sulphide, dioctyl sulphide, propyl 4-methylcyclohexyl sulphide or methyl 4-chlorocyclohexyl sulphide.

**7.** Process according to Claim 5 or 6, characterised in that the chlorine-accepting reducing compound used is thioanisole.

**8.** Process according to either of Claims 1 and 2, characterised in that the chlorine-accepting reducing compound is chosen from the thiols, mercaptoacids and thiocarboxylic acids of general formula (III):

$R_7$ - SH (III)

in which $R_7$ represents:
- a linear or branched alkyl radical, optionally substituted by one or more OH groups, -COOH groups or chlorine atoms and which can contain in its chain one or more -O- oxygen atoms;
- a phenyl radical, optionally substituted by one or more alkyl, alkoxy, hydroxyl or carboxyl radicals or chlorine atoms;
- a phenylalkyl radical, optionally substituted by one or more alkyl, alkoxy, hydroxyl or carboxyl radicals or chlorine atoms;
- a cyclopentyl or cyclohexyl radical, optionally substituted by one or more alkyl, alkoxy, hydroxyl or carboxyl radicals or chlorine atoms;
- a radical $R_7$-CO- in the formula of which $R_7$ has the meanings indicated above

**9.** Process according to Claim 8, characterised in that the chlorine-accepting reducing compound used is chosen from 2-mercaptoethanol, 3,4-toluenedithiol, 2-methyl-2-propanethiol, thiophenol, thioacetic acid, thioglycolic acid or 2-mercaptosuccinic acid.

**10.** Process according to either of Claims 1 and 2, characterised in that the chlorine-accepting reducing compound is chosen from alkali metal, alkaline-earth metal or aluminium and alkali metal hydrides; alkali metal borohydrides; alkali metal cyanoborohydrides and alkali metal alkoxyborohydrides.

**11.** Process according to Claim 10, characterised in that the chlorine-accepting reducing compound is chosen from sodium hydride, potassium hydride, lithium hydride, calcium hydride, magnesium hydride, lithium aluminium hydride, sodium borohydride, potassium borohydride, sodium cyanoborohydride, potassium cyanoborohydride, sodium trimethoxyborohydride, sodium triethoxyborohydride, potassium trimethoxyborohydride, potassium triethoxyborohydride or sodium dimethoxyborohydride.

**12.** Process according to either of Claims 1 and 2, characterised in that the chlorine-accepting reducing compound is chosen from copper, stannous chloride or ruthenium trichloride.

**13.** Process according to either of Claims 1 and 2, characterised in that the chlorine-accepting reducing compound is chosen from phenols, diphenols and triphenols and these compounds which contain one or more alkyl or alkoxy substituents having 1 to 4 carbon atoms.

**14.** Process according to Claim 13, characterised in that the chlorine-accepting reducing compound is chosen from phenol, 2-methylphenol, 3-methylphenol, 4-methylphenol, 2-methoxyphenol, 3-methoxyphenol, 4-methoxyphenol, 2-ethoxyphenol, 3-ethoxyphenol, 4-ethoxyphenol, 4-(tert-butyl)phenol, pyrocatechol, resorcinol, hydroquinone, 2-methylhydroquinone, 2-methoxyhydroquinone, 4-methoxypyrocatechol, 2,6-dimethoxyhydroquinone, 1-naphthol, 2-naphthol, 1,2-dihydroxynaphthalene, 1,3-dihydroxynaphthalene, 1,4-dihydroxynaphthalene, 1,5-dihydroxynaphthalene, 1,6-dihydroxynaphthalene, 1,7-dihydroxynaphthalene, 1,8-dihydroxynaphthalene, 2,3-dihydroxynaphthalene, 2,6-dihydroxynaphthalene, 2,7-dihydroxynaphthalene, pyrogallol, phloroglucinol, hydroxyquinol, 2-chlorohydroquinone or vitamin E.

**15.** Process according to one of Claims 1, 13 and 14, characterised in that phosphorus trichloride, phosphorous acid or hypophosphorous acid is introduced into the mixture to be treated.

**16.** Process according to one of Claims 1 to 15, characterised in that the process is carried out at a temperature from 20 ° C to 200 ° C and preferably from 60 to 150 ° C.

**17.** Process according to one of Claims 1 to 16, characterised in that a molar quantity of chlorine-accepting reducing compound which is at least equal to the molar quantity of the gem-dichlorinated cyclic ketones of the mixture to be treated is used and preferably a molar excess of chlorine-accepting reducing compound is used.

**Patentansprüche**

**1.** Verfahren zur Stabilisierung einer Mischung, die von der Chlorierung von Phenol und/oder Chlorphenolen stammt, dadurch gekennzeichnet, daß man die Mischung in Gegenwart zumindest einer reduzierenden Chlorakzeptorverbindung rührt.

**2.** Verfahren nach Anspruch 1, dadurch gekennzeichnet, daß die reduzierende Chlorakzeptorverbindung ausgewählt ist aus den organischen Phosphiten; den Phosphinen; den Phosphiniten; den Arsinen, den organischen Sulfiden; den Thiolen, den Thiocarbonsäuren; den Mercaptosäuren; den Metallhydriden; den Metallborhydriden; den Metallcyanoborhydiden; den Alkoxyborhydriden, gewissen Metallen wie Kupfer, Zinn, Silber, Kobalt, Palladium, Platin, Iridium, Germanium und Selen oder Derivaten von gewissen dieser Metalle; den Phenolen, Diphenolen und Triphenolen.

**3.** Verfahren nach einem der Ansprüche 1 oder 2, dadurch gekennzeichnet daß die reduzierende Chlorakzeptorverbindung eine Verbindung von dreiwertigem Phosphor der allgemeinen Formel (I) ist:

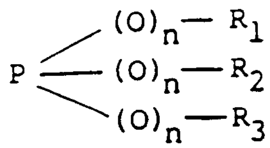

(I)

worin

- $R_1$, $R_2$ und $R_3$ unabhängig voneinander darstellen:
  - einen geradkettigen oder verzweigten Alkylrest mit 1 bis 12 Kohlenstoffatomen;
  - einen geradkettigen oder verzweigten Alkylrest mit 1 bis 12 Kohlenstoffatomen, der ein bis mehrere Chloratome trägt;
  - einen Alkylrest, der eine oder mehrere Etherfunktionen, z.B. insbesonders eine oder mehrere Ethylenoxid- und/oder Propylenoxidketten, trägt;
  - einen Phenylrest;
  - einen Phenylrest, der ein oder mehrere Chloratome trägt;
  - einen Phenylrest, der 1 oder 2 Alkylsubstituenten mit 1 bis 12 Kohlenstoffatomen trägt,
  - einen Phenylalkylrest, dessen aliphatischer Teil 1 bis 4 Kohlenstoffatome trägt und dessel cyclischer Teil ein oder mehrere Chloratome oder 1 oder 2 Alkylreste mit 1 bis 12 Kohlenstoffatomen tragen kann;
  - einen Cycloalkyl-, insbesonders Cyclohexylrest, gegebenenfalls substituiert durch ein oder mehrere Chloratome oder 1 oder 2 Alkylreste mit 1 bis 12 Kohlenstoffatomen;
- ein oder zwei Reste $R_1$, $R_2$ und $R_3$ ein Wasserstoffatom darstellen können;
- die Symbole n, die gleich oder voneinander verschieden sind, 0 oder 1 darstellen.

**4.** Verfahren nach Anspruch 3, dadurch gekennzeichnet daß die Verbindung von dreiwertigem Phosphor der Formel (I) ausgewählt ist aus Triphenylphosphit, den Tris(chlorphenyl)phosphiten, den Tris-(dichlorphenyl)phosphiten, den Tris(trichlorphenyl)phosphiten, Trimethylphosphit, Triethylphosphit, Tributylphosphit, Trioctylphosphit, den Tris(nonylphenyl)phosphiten, Tris(2,4-ditertiobutylphenyl)phosphit, Tribenzylphosphit, Tris(phenethyl)phosphit, Methyldiphenylphosphinit, Tris(2-ethoxy-ethyl)phosphit, Tricyclohexylphosphit, Triphenylphosphin, Diphenylmethylphosphin, Diphenylethylphosphin, Diethylphenylphosphin, Tributylphosphin.

**5.** Verfahren nach einem der Ansprüche 1 oder 2, dadurch gekennzeichnet, daß die reduzierende Chlorakzeptorverbindung ausgewählt ist aus den organischen Sulfiden der allgemeinen Formel (II):

$R_5$ - S - $R_6$ (II)

in welcher $R_5$ und $R_6$ unabhängig voneinander darstellen:

- einen Phenylrest, gegebenenfalls substituiert durch einen oder mehrere Alkyl-, Alkoxy-, Hydroxylreste oder Chloratome;
- einen Alkylrest;
- einen Phenylalkylrest, gegebenenfalls substituiert durch einen oder mehrere Alkyl-, Alkoxy-, Hydroxylreste oder Chloratome;
- einen Cyclopentyl- oder Cyclohexylrest, gegebenenfalls substituiert durch einen oder mehrere Alkyl-, Alkoxy-, Hydroxylreste oder Chloratome.

**6.** Verfahren nach Anspruch 5, dadurch gekennzeichnet, daß die reduzierende Chlorakzeptorverbindung ausgewählt ist aus Thioanisol, Diphenylsulfid, Phenyl- und 4-Chlorphenylsulfid, Di(4-chlorphenyl)sulfid, Di(2-hydroxy-4,6-ditertiobutylphenyl)sulfid,Di(4-hydroxy-3,5-dimethylphenyl)sulfid, Di(2-hydroxy-3,5-ditertiobutylphenyl)sulfid, Phenyl- und Cyclohexylsulfid, Phenyl- und Butylsulfid, 4-Chlorphenyl- und Hexylsulfid, 4-Methylphenyl- und Dodecylsulfid, Benzyl- und Octylsulfid, 4-Methoxyphenyl- und Ethylsulfid, Phenyl- und Stearylsulfid, Dihexylsulfid, Ethyl- und Hexylsulfid, Dioctylsulfid, Propyl- und 4-Methylcyclohexylsulfid, Methyl- und 4-Chlorcyclohexylsulfid.

**7.** Verfahren nach den Ansprüchen 5 oder 6, dadurch gekennzeichnet, daß die verwendete reduzierende Chlorakzeptorverbindung Thioanisol ist.

8. Verfahren nach einem der Ansprüche 1 oder 2, dadurch gekennzeichnet, daß die reduzierende Chlorakzeptorverbindung ausgewählt ist aus den Thiolen, den Mercaptosäuren und den Thiocarbonsäuren der allgemeinen Formel (III):

$R_7$ - SH (III)

in welcher $R_7$ darstellt:
- einen geradkettigen oder verzweigten Alkylrest, gegebenenfalls substituiert durch eine oder mehrere OH-Gruppen, -COOH-Gruppen oder Chloratome, der in seiner Kette ein oder mehrere Sauerstoffatome -O- tragen kann;
- einen Phenylrest, gegebenenfalls substituiert durch einen oder mehrere Alkyl-, Alkoxy-, Hydroxyl-, Carboxylreste oder Chloratome;
- einen Phenylalkylrest, gegebenenfalls substituiert durch einen oder mehrere Alkyl-, Alkoxy-, Hydroxy-, Carboxylreste oder Chloratome;
- einen Cyclopentyl- oder Cyclohexylrest, gegebenenfalls substituiert durch einen oder mehrere Alkyl-, Alkoxy-, Hydroxyl-, Carboxylreste oder Chloratome;
- einen Rest $R_7$-CO-, in dessen Formel $R_7$ die zuvor angegebenen Bedeutungen hat.

9. Verfahren nach Anspruch 8, dadurch gekennzeichnet, daß die verwendete reduzierende Chlorakzeptorverbindung ausgewählt ist aus 2-Mercaptoethanol, 3,4-Toluoldithiol, 2-Methyl-2-propanthiol, Thiophenol, Thioessigsäure, Thioglycolsäure, 2-Mercaptobernsteinsäure.

10. Verfahren nach einem der Ansprüche 1 oder 2, dadurch gekennzeichnet, daß die reduzierende Chlorakzeptorverbindung ausgewählt ist aus den Alkalimetall-, Erdalkalimetall-, oder Aluminium- und Alkalimetallhydriden; den Alkalimetallborhydriden; den Alkalimetallcyanoborhydriden und den Alkalimetallalkoxyborhydriden.

11. Verfahren nach Anspruch 10, dadurch gekennzeichnet, daß die reduzierende Chlorakzeptorverbindung ausgewählt ist aus Natriumhydrid, Kaliumhydrid, Lthiumhydrid, Calciumhydrid, Magnesiumhydrid, Aluminium- und Lithiumhydrid, Natriumborhydrid, Kaliumborhydrid, Natriumcyanoborhydrid, Kaliumcyanoborhydrid, Natriumtrimethoxyborhydrid, Natriumtriethoxyborhydrid, Kaliumtrimethoxyborhydrid, Kaliumtriethoxyborhydrid, Natriumdimethoxyborhydrid.

12. Verfahren nach einem der Ansprüche 1 oder 2, dadurch gekennzeichnet, daß die reduzierende Chlorakzeptorverbindung ausgewählt ist aus Kupfer, Zinn(II)chlorid, Rutheniumtrichlorid.

13. Verfahren nach einem der Ansprüche 1 oder 2, dadurch gekennzeichnet, daß die reduzierende Chlorakzeptorverbindung ausgewählt ist aus den Phenolen, den Diphenolen und den Triphenolen und diesen Verbindungen, die einen oder mehrere Alkyl-und Alkoxysubstituenten mit 1 bis 4 Kohlenstoffatomen enthalten.

14. Verfahren nach Anspruch 13, dadurch gekennzeichnet, daß die reduzierende Chlorakzeptorverbindung ausgewählt ist aus Phenol, 2-Methylphenol, 3-Methylphenol, 4-Methylphenol, 2-Methoxyphenol, 3-Methoxyphenol, 4-Methoxyphenol, 2-Ethoxyphenol, 3-Ethoxyphenol, 4-Ethoxyphenol, 4-Tertiobutylphenol, Pyrokatechol, Resorcinol, Hydrochinon, 2-Methylhydrochinon, 2-Methoxyhydrochinon, 4-Methoxypyrokatechol 2,6-Dimethoxy-hydrochinon, 1-Naphthol, 2-Naphthol, 1,2-Dibydroxynaphthalin, 1,3-Dihydroxynaphthalin, 1,4-Dihydroxynapilthalin, 1,5-Dihydroxynaphthalin, 1,6-Dihydroxynaphthalin, 1,7-Dihydroxynaphthalin, 1,8-Dihydroxynaphthalin, 2,3-Dihydroxynaphthalin, 2,6-Dihydroxynaphthalin, 2,7-Dihydroxynaphthalin, Pyrogallol, Phloroglucin , Hydroxychinol, 2-Chlorhydrochinon, Vitamin E.

15. Verfahren nach einem der Ansprüche 1, 13 und 14, dadurch gekennzeichnet, daß man in die zu behandelnde Mischung Phosphortrichlorid, phosphorige oder hypophosphorige Säure einbringt.

16. Verfahren nach einem der Ansprüche 1 bis 15, dadurch gekennzeichnet, daß man bei einer Temperatur von 20 ° C bis 200 ° C und vorzugsweise von 60 bis 150 ° C arbeitet.

17. Verfahren nach einem der Ansprüche 1 bis 16, dadurch gekennzeichnet, daß man eine molare Menge an reduzierender Chlorakzeptorverbindung, die zumindest gleich der molaren Menge der *gem*-dichlorierten cyclischen Ketone der zu behandelnden Mischung ist, und vorzugsweise einen molaren Überschuß an reduzierender Chlorakzeptorverbindung verwendet.